# EUROPEAN PATENT APPLICATION

(11) **EP 4 582 480 A1**
(43) Date of publication of application: **09.07.2025**
(21) Application number: 23935897.1
(22) Date of filing: 10.11.2023
(51) Int. Cl.: C08L 69/00, C07D 207/00, C07D 221/00, C07D 265/00, C07D 309/00

(54) **BLUE-VIOLET LIGHT-ABSORBING COMPOSITION COMPRISING PHENYLACRYLONITRILE COMPOUND, PREPARATION METHOD THEREFOR, AND PRODUCT COMPRISING SAME**

(71) Applicant: Chitec Technology Co., Ltd., Taipei City 110502 (TW)
(72) Inventor: CHANG, Wei-Chun, Taipei City 110502, Taiwan (TW); WU, Huang-Min, Taipei City 110502, Taiwan (TW); CHENG, Ching-Hao, Taipei City 110502, Taiwan (TW); HUANG, Yi-Shuo, Taipei City 110502, Taiwan (TW); WU, Chi-Feng, Taipei City 110502, Taiwan (TW); LUO, De-Shun, Taipei City 110502, Taiwan (TW); CHEN, Si-Yuan, Taipei City 110502, Taiwan (TW); CHIANG, Yen-Hei, Taipei City 110502, Taiwan (TW); SUNG, Wei-Cheng, Taipei City 110502, Taiwan (TW)
(74) Representative: Becker, Erin
(86) International application number: PCT/CN2023/130898
(87) International publication number: WO 2025/097414

(57) **Abstract**

The present disclosure relates to a composition comprising a phenylacrylonitrile compound of formula (I) for the use in absorbing blue-violet light, the method of producing the same, and the product comprising the same.

## Description

### FIELD OF INVENTION

The present invention relates to a blue-violet light absorbing composition comprising phenylacrylonitrile, which can be used in the fields such as optical films and coatings.

### BACKGROUND OF THE INVENTION

Visible light is divided into red, orange, yellow, green, indigo, and violet light, in which the wavelength of red light is the longest and the wavelength of violet light is the shortest. The shorter the wavelength, the higher the energy. A study report by a German ophthalmologist, Dr. R. H. W. Funk, indicated that continued irradiation of "improper light" to eyes would cause dysfunction. Particularly, trichromatic lamps or computer screens emit a large number of high-energy short-wavelength blue-violet light with irregular frequencies. The short-wavelength blue-violet light has higher energy and can transmit through the crystalline lens to the retina, and would impose photochemical damage to the retina, cause direct or indirect damage to cells in the area of macula lutea, and result in macular degeneration in the long term.

An excellent blue-violet absorber must have two essential properties: 1. it can filter blue-violet light and 2. it has a light color. In addition, it also needs to have 3. properties of high-temperature processing and high-temperature environment resistance, and 4. good life time. In the high-end applications, the absorbance of blue-violet absorbers to particular blue light bands would gradually decrease with the increase of wavelength, and blue light with longer wavelength must have greater transmittance. For example, in the wavelength range of 420 nm to 460 nm, the blue light transmitted by optical lens need to have a gradually increasing transmittance of from 50% to 100%, so as to result in better visual perception. Hence, the condition for producing a blue-violet absorber is highly strict. Further, adding blue-violet absorber to plastics requires high temperature processing or use outdoor at high temperature. Thus, a property of resistance to high temperature processing is a very important requirement.

### SUMMARY OF THE INVENTION

The present disclosure relates to a blue-violet light absorbing composition comprising a specific structure of phenylacrylonitrile, which can be used in the fields such as optical films and coatings. The blue-violet light absorbing composition can be processed and prepared into optical films and coatings to absorb light for eye protection, and can also selectively absorb long wavelength blue light for the optimal visual effect of transmitted light. The composition according to the present disclosure has the special advantages of light color and absorbing blue-violet light, and can be utilized in the technical fields such as plastics, coating materials, ink, display devices, lighting devices, optical films, optical lens, glasses, textiles, pressure sensitive adhesives, or sunscreen products; particularly in the screen protecting films of mobile phones, computers, or televisions or in glasses industry and thus have potential application prospects.

An aspect of the present disclosure is a blue-violet light absorbing composition, comprising a polymer and a blue-violet light absorber, wherein the blue-violet light absorber is a compound of the following Formula (I): 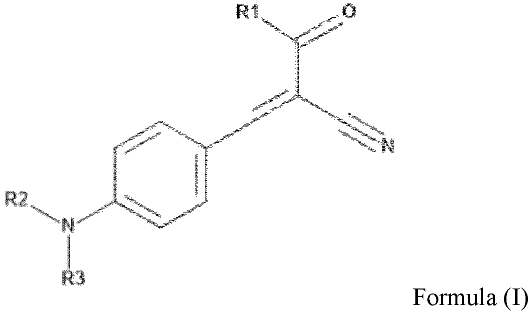 wherein R₁ is a five-membered or six-membered heterocycloalkyl group comprising 1 or 2 heteroatom(s) selected from the group consisting of oxygen and nitrogen and is unsubstituted or substituted with at least one of C₁₋₈ alkyl, -OH, -N=O, -CN, or halogen; and R₂ and R₃ are independently selected from H or C₁₋₈ alkyl.

In one embodiment, R₁ is selected from the group consisting of unsubstituted or substituted pyrrolidinyl, tetrahydrofuranyl, oxazolidinyl, isoxazolidinyl, tetrahydropyranyl, piperidinyl, morpholinyl, 1,2-oxazinanyl, and 1,3-oxazinanyl.

In one embodiment, R₁ is selected from 4-morpholinyl, 1-piperidinyl, or pyrrolidin-1-yl; R₂ is selected from H, methyl, or ethyl; and R₃ is selected from H, methyl, or ethyl.

In one embodiment, the blue-violet light absorber is selected from one or any combination of the following compounds:

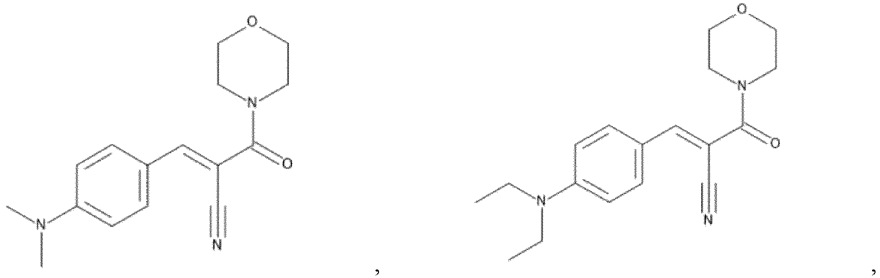

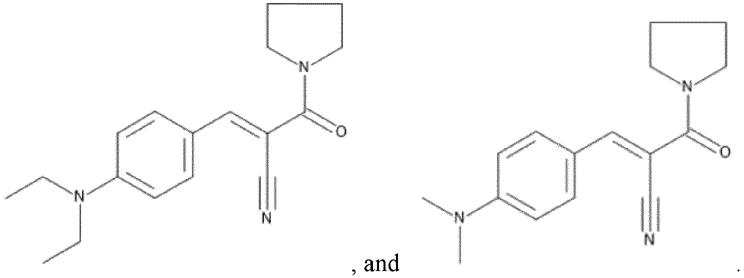

In one embodiment, the polymer is a curable transparent or semitransparent polymer.

In one embodiment, the polymer is selected from the group consisting of: cellulose ester, polyamide, polyimide, polyurethane, epoxy resin, amino resin, polycarbonate, polyester, polyolefin, acrylic resin, polyformaldehyde, polysulfone, polyethersulfone, polyesterketone, polyether imide, polyoxyethylene, silicone, liquid crystal polymer, and any combination thereof.

In one embodiment, the blue-violet light composition further comprises one or more additional additive selected from the group consisting of: antistatic agent, antifoaming agent, levelling agent, wetting agent, thickening agent, dispersing agent, wax, matting agent, antimicrobial agent, metal oxide screening agent, photostabilizing agent, thermostabilizing agent, antioxidant, peroxide scavenger, free radical scavenger, filler, rubber, preservative, fire retardant, plasticizer, dye, pigment, brightener, fluorescent whitening agent, antiager, metal stabilizer, acid scavenger, antihydrolysis agent, and any combination of at least two of the aforesaid additives.

In one embodiment, the blue-violet light composition further comprises one or more light absorbers selected from the group consisting of infrared absorber, ultraviolet absorber, blue light absorber, and any combination of at least two of the aforesaid absorbers.

In one embodiment, the amount of the blue-violet light absorber is about 0.01% to about 20% of the total weight of the blue-violet light absorbing composition.

Another aspect of the present disclosure is a method of preparing a blue-violet light absorbing composition, comprising, mixing a polymer and a blue-violet light absorber of the Formula (I) according to the present disclosure to obtain a blue-violet light absorbing composition. In one embodiment, 0.01 to 20 parts by weight of the blue-violet light absorber is mixed with 80 to 100 parts by weight of the polymer.

In one embodiment, the method further comprises heating the polymer to 200 °C to 280 °C for melting, and cooling for curing.

Another aspect of the present disclosure is a blue-violet light absorbing product, comprising the blue-violet light absorbing composition according to the present disclosure.

In one embodiment, the blue-violet light absorbing product is selected from the group consisting of plastics, coating material, ink, sunscreen, display device, lighting device, optical film, optical lens, glass, textile, and pressure sensitive adhesive.

Another aspect of the present disclosure is a method of preparing blue-violet light absorbing product, comprising: providing the blue-violet light absorbing composition of the present disclosure, and disposing the blue-violet light absorbing composition on an article to obtain the blue-violet light absorbing product.

In one embodiment, the blue-violet light absorbing composition is processed via a method selected from the group consisting of dip coating, granulation, extrusion, lamination, injection molding, calendaring, casting, film blowing, coating, melt blowing, spinning, or any combination thereof.

### DETAILED DESCRIPTION

The features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, as described below. Moreover, as the contents disclosed herein should be readily understood and can be implemented by a person skilled in the art, all equivalent changes or modifications which do not depart from the concept of the present disclosure should be encompassed by the appended claims.

Unless defined otherwise, all technical and scientific terms in the specification and claims can be appreciated by a skilled person in the technical field of the present disclosure. The singular forms "a," "an", "the", or the like include plural referents unless the context clearly dictates otherwise. In this specification, the use of "or" or "and" means "and/or" unless stated otherwise. Furthermore, use of the term "including" as well as other forms, such as "include", "includes," and "included," is not limiting. The section headings used herein are for organizational purposes only and are not to be construed as limiting the subject matter described. Unless otherwise stated, the materials in the present disclosure can be obtained commercially.

Any numerical value such as concentration or concentration range described herein is to be understood as being modified in all instances by the term "about." The term "about" means within an acceptable error range for the particular value as determined by one of ordinary skill in the art, which will depend in part on how the value is measured or determined, i.e., the limitations of the measurement system. Unless explicitly stated otherwise within the Examples or elsewhere in the Specification in the context of a particular assay, result or embodiment, the term "about" means within one standard deviation per the practice in the art, or a range of up to 1%, 2%, 3%, 4%, or 5%, whichever is larger.

According to the present disclosure, the term "alkyl" refers to unbranched saturated hydrocarbon chain or branched saturated hydrocarbon chain. As used herein, the alkyl has 1 to 8 carbon atoms (i.e., C₁-C₈ alkyl), 1 to 6 carbon atoms (i.e., C₁-C6 alkyl), 1 to 4 carbon atoms (i.e., C₁-C₄ alkyl), or 1 to 3 carbon atoms (i.e., C₁-C₃ alkyl). The examples of alkyl include methyl, ethyl, propyl, isopropyl, n-butyl, sec-butyl, isobutyl, tert-butyl, pentyl, 2-pentyl, isopentyl, neopentyl, hexyl, 2-hexyl, 3-hexyl, 3-methylpentyl, 2-ethylhexyl, heptyl, isoheptyl, octyl, or isooctyl. While an alkyl substitution with particular carbon number is determined by nomenclature or molecular formula, it includes all the isomer with the same carbon number; as such, for example, "propyl" refer to n-propyl (i.e., -(CH₂)₂CH₃) or isopropyl (i.e., - CH(CH₃)₂); and "butyl" refers to n-butyl (i.e., -(CH₂)₃CH₃), isobutyl (i.e., - CH₂CH(CH₃)₂), sec-butyl (i.e., -CH(CH₃)CH₂CH₃), or tert-butyl (i.e., -C(CH₃)₃).

As used herein, the term "heterocycloalkyl" refers to a saturated cycloalkyl having one or more heteroatom(s) independently selected from nitrogen and oxygen. The heterocycloalkyl group can have one or more substituent, e.g., selected from alkyl, -OH, -N=O, -ONH₂, CN, or halogen. The heterocycloalkyl group can be a five-membered or six-membered heterocycloalkyl group, with one nitrogen heteroatom, or with one oxygen heteroatom, or with one nitrogen heteroatom and one oxygen heteroatom. Hence, according to the present disclosure, the heterocycloalkyl group includes a cyclic structure with 1 nitrogen and 4 carbons, with 1 oxygen and 4 carbons, with 1 nitrogen and 1 oxygen and 3 carbons, with 1 nitrogen and 5 carbons, with 1 oxygen and 5 carbons, or 1 nitrogen and 1 oxygen and 4 carbons.

The term "halogen" *per se* or as a part of other group refers to fluorine, chlorine, bromine, or iodine.

The term "cyano" refers to the group "-CN".

As used herein, the term "optionally" means that the event or condition subsequently illustrated occurs or does not occur. The term "optionally substitute" means that one or more hydrogen on particular atom or group is not substituted or is substituted by a moiety other than hydrogen. For example, the compound of Formula (I) of the present disclosure has optionally substituted five-membered or six-membered heterocycloalkyl R₁, and optionally substituted R₂ and R₃.

A substituted group can be substituted by one or more substituent(s) (such as 1, 2, 3, 4, or 5 substituents). In some embodiments, the substituent(s) is selected from the functional groups provided herein. In some embodiments, the substituent is selected from C₁₋₈ alkyl, -OH, -N=O, -ONH₂, CN, or halogen.

In some embodiments, the substituent is C₁₋₈ alkyl, including but not limited to methyl, ethyl, propyl, isopropyl, n-butyl, sec-butyl, isobutyl, tert-butyl, pentyl, 2-pentyl, isopentyl, neopentyl, hexyl, 2-hexyl, 3-hexyl, 3-methylpentyl, 2-ethylhexyl, heptyl, isoheptyl, octyl, or isooctyl.

In some embodiments, the substituent is halogen such as fluorine, chlorine, bromine, or iodine.

Unless indicated otherwise, the compound of the present disclosure comprises all possible geometric isomers, for example, Z and E isomers (cis- and trans-isomers), and all possible optical isomers, for example, diastereomers and enantiomers. Therefore, the compounds of the embodiments according to the present disclosure include the geometric isomers and optical isomers thereof. Further, the scope of disclosure includes individual isomers or the mixture thereof, for example, racemic mixture. Individual isomer can be obtained from a corresponding isomer of a staring material, or can be isolated by a conventional isolation method after preparing a final compound. Conventional resolution method such as fractional crystallization method can be employed to isolate optical isomers such as enantiomers from a mixture.

The term used herein "curable transparent or semitransparent polymer" refers to a polymer presented as transparent or semitransparent after curing. The term "transparent polymer" refers to a polymer that almost does not absorb any visible light; the term "semitransparent polymer" refers to a polymer that only reduces the transmittance of visible light.

An aspect of the present disclosure provides a blue-violet light absorbing composition, comprising a polymer and a blue-violet light absorber, wherein the blue-violet light absorber is a phenylacrylonitrile compound of the following Formula (I): 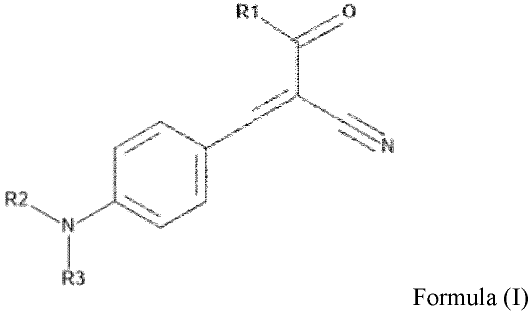 wherein
R₁ is a five-membered or six-membered heterocycloalkyl group comprising 1 or 2 heteroatom(s) selected from the group consisting of oxygen and nitrogen and is unsubstituted or substituted with at least one of C₁₋₈ alkyl, -OH, -N=O, -CN, or halogen; and
R₂ and R₃ are independently selected from H or C₁₋₈ alkyl.

Accordingly, the hetero cyclic structure of R₁ comprises one oxygen atom, or comprises one nitrogen atom, or comprises one oxygen atom and one nitrogen atom.

In one embodiment, R₁ is selected from the group consisting of unsubstituted or substituted pyrrolidinyl, tetrahydrofuranyl, oxazolidinyl, isoxazolidinyl, tetrahydropyranyl, piperidinyl, morpholinyl, 1,2-oxazinanyl, and 1,3-oxazinanyl.

In one embodiment, R₁ is selected from the group consisting of substituted or unsubstituted pyrrolidinyl, piperidinyl, and morpholinyl.

In one embodiment, R₁ is selected from the group consisting of pyrrolidin-1-yl, 1-piperidinyl, and 4-morpholinyl.

According to the present disclosure, C₁₋₈ alkyl refers to branched or unbranched C₁₋₈ alkyl, the examples of which include but is not limited to methyl, ethyl, propyl, isopropyl, n-butyl, sec-butyl, isobutyl, tert-butyl, n-pentyl, 2-pentyl, 3-pentyl, neopentyl, hexyl, 2-hexyl, 3-hexyl, 3-methylpentyl, 2-ethylhexyl, heptyl, isoheptyl, octyl, or isooctyl.

In a particular embodiment, C₁₋₈ alkyl comprises branched or unbranched C₁₋₄ alkyl, for example, methyl, ethyl, propyl, isopropyl, n-butyl, sec-butyl, isobutyl, or tert-butyl. In a particular embodiment, C₁₋₈ alkyl is methyl or ethyl.

Therefore, in one embodiment, R₁ is a five-membered or six-membered heterocycloalkyl substituted by C₁₋₈ alkyl, for example, substituted by at least one of methyl, ethyl, propyl, isopropyl, n-butyl, sec-butyl, isobutyl, tert-butyl, n-pentyl, 2-pentyl, 3-pentyl, neopentyl, hexyl, 2-hexyl, 3-hexyl, 3-methylpentyl, 2-ethylhexyl, heptyl, isoheptyl, octyl, or isooctyl.

In one embodiment, the cyclic structure of R₁ is substituted by halogen, wherein the halogen is selected from fluorine, chlorine, bromine, or iodine.

In one embodiment, the ring structure of R₁ does not have a substituent. In another embodiment, the ring structure of R₁ has at least one substituent, for example, has one, two, or three substituents.

In one embodiment, R₂ and R₃ are identical or different as H or C₁₋₈ alkyl. For example, R₂ and R₃ are independently selected from the group consisting of methyl, ethyl, propyl, isopropyl, n-butyl, sec-butyl, isobutyl, tert-butyl, n-pentyl, 2-pentyl, 3-pentyl, neopentyl, hexyl, 2-hexyl, 3-hexyl, 3-methylpentyl, 2-ethylhexyl, heptyl, isoheptyl, octyl, or isooctyl.

In one embodiment of the compound of Formula (I), R₁ is unsubstituted or is substituted by a five-membered or six-membered heterocycloalkyl group which is substituted by at least one methyl or ethyl, and R₂ and R₃ are respectively H, methyl, or ethyl.

In one embodiment, the blue-violet light absorbing composition comprises a polymer and a blue-violet light absorber, wherein the blue-violet light absorber is the compound Formula (I) according to the present disclosure, wherein R₁ is selected from 4-morpholinyl, 1-piperidinyl, or pyrrolidin-1-yl; and R₂ and R₃ are respectively selected from H, methyl, or ethyl.

According to the present disclosure, the compound of Formula (I) comprises the cis-trans isomers or optical isomers thereof. In one embodiment, the cis-trans isomers of the compound of Formula (I) comprises the isomers of Formula (Ia) or Formula (Ib):

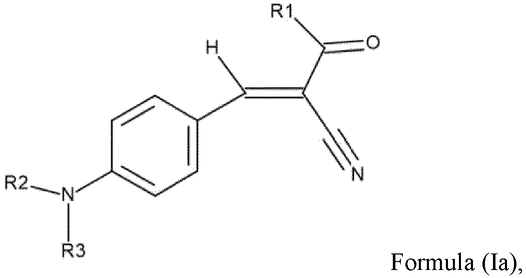

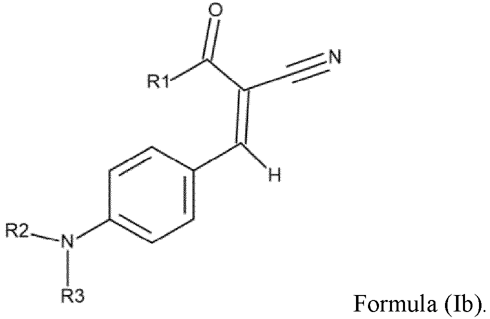

In a particular embodiment, the compound Formula (I) is selected from any of the following compounds or the isomers thereof:

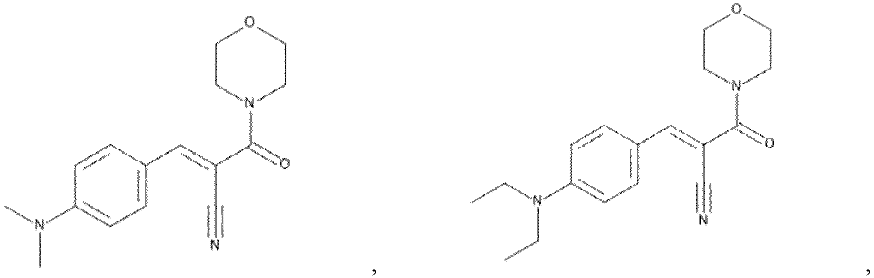

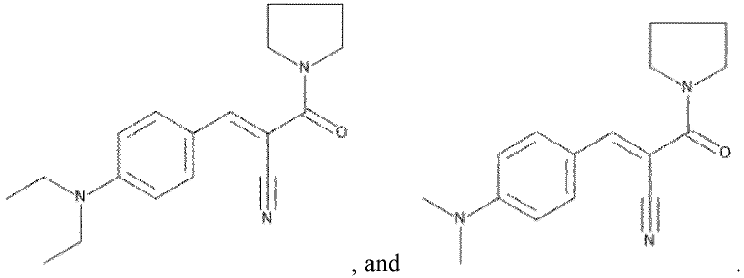

In one embodiment, the blue-violet light absorber according to the present disclosure comprises at least one compound encompassed by Formula (I) at any ratio; for example, the blue-violet light absorber comprises one, two, three, four, or more compound(s) encompassed by Formula (I).

In one embodiment, the blue-violet light absorber absorbs the blue-violet light with wavelength range from 380 nm to 460 nm, particularly, the blue-violet light with wavelength range from 380 nm to 450 nm, more preferably 380 nm to 440 nm, and particularly preferably 390 nm to 420 nm.

In one embodiment, the polymer is a curable transparent or semitransparent polymer. Preferably, the refractive index of the polymer after curing is at least 1.45, preferably at least 1.5, or more preferably at least 1.6. Preferably, the transmittance of the transparent or semitransparent polymer is more than 80%, preferably more than 90%, more preferably more than 95%, or most preferably more than 99%. In addition, the haze of the polymer is preferably less than 2%, or more preferably less than 1%.

The polymer is preferably dissolved in a solvent. If the polymer is a curable polymer and is dissolved in a solvent, when it is coated on a substrate, the solvent can be removed and the polymer is cured to form a cured blue-violet light absorbing composition. Alternatively, the polymer is melted and liquefied, and is mixed with the blue-violet light absorber, and then is cooled to form a cured polymer.

The transparent or semitransparent polymer used in the present disclosure includes, for example, inorganic materials, e.g., cellulose esters, such as diacetyl cellulose, triacetyl cellulose (TAC), propionyl cellulose, butyryl cellulose, acetylpropionyl cellulose, and nitrocellulose; polyamides; polyimides; polyurethanes; epoxy resins; polycarbonates; polyesters, such as polyethylene terephthalate (PET), polyethylene naphthalate (PEN), polybutylene terephthalate (PBT), poly(1,4-cyclohexane dimethylene terephthalate), and poly(ethylene-1,2-diphenoxyethane-4,4'-dicarboxylate); polyolefins, such as polystyrene, polyethylene, polypropylene, polymethylpentene (PMP), and acrylonitrile-butadiene-styrene copolymer (ABS); polyvinyl compounds, such as polyvinyl acetate, polyvinyl chloride, and polyvinyl fluoride; acrylic resins, such as polymethacrylate ester, polymethyl methacrylate, and polyacrylate copolymer; polyformaldehyde, polysulfones; polyether sulfones; polyether ketones; polyether imides (PEI); polyoxyethylenes; silicone, liquid crystal polymer, or any combination thereof.

Thus, in one embodiment, the polymer is selected from cellulose ester, polyamide, polyimide, polyurethane, epoxy resin, amino resin, polycarbonate, polyester, polyolefin, acrylic resin, polyformaldehyde, polysulfone, polyethersulfone, polyesterketone, polyether imide, polyoxyethylene, silicone, liquid crystal polymer, or any combination thereof.

In a preferred embodiment, the polymer is selected from the group consisting of polyurethane, polycarbonate, acrylic resin, and any combination thereof.

In one embodiment, the blue-violet light absorbing composition according to the present disclosure further comprises one or more additional additive(s) selected from antistatic agent (e.g., graphene or nanocarbon tube), antifoaming agent, levelling agent, wetting agent, thickening agent, dispersing agent, wax, matting agent, antimicrobial agent, metal oxide screening agent, photostabilizing agent, thermostabilizing agent, antioxidant (e.g., phenol, phosphorus-containing antioxidant, or thioether antioxidant), peroxide scavenger, free radical scavenger, filler, rubber (e.g., silicone rubber), preservative, fire retardant, plasticizer, dye, pigment (titanium white or carbon black, etc.), brightener, fluorescent whitening agent, antiager, metal stabilizer, acid scavenger, antihydrolysis agent, and any combination of at least two of the aforesaid additives.

In one embodiment, the blue-violet light absorbing composition comprises additional absorbers, for example, infrared absorbers, ultraviolet light absorbers, blue light absorbers, or other colorants.

The examples of ultraviolet absorbers include but not limited to triazine-based compounds, benzotriazole-based compounds, diphenylketone-based compounds, merocyanine-based compounds, cyanine-based compounds, dibenzoylmethane-based compounds, cinnamic acid-based compounds, cyanoacrylate-based compounds, and benzoate-based compounds.

The more specific examples of the ultraviolet absorbers include, for example, 2-(2'-hydroxyphenyl)benzotriazole, 2-(2'-hydroxy-5'-methylphenyl)benzotriazole, 2-(2'-hydroxy-3',5'-di-tert-butylphenyl)-5-chlorobenzotriazole, 2-(2'-hydroxy-3'-tert-butyl-5'-methylphenyl)-5-chlorobenzotriazole, 2-(2'-hydroxy-5'-tert-octylphenyl)benzotriazole, 2-(2'-hydroxy-3',5'-dicumylphenyl)benzotriazole, 2-(2'-hydroxy-3'-tert-butyl-5'-carboxyphenyl)benzotriazole, 2,2'-methylenebis(4-tert-octyl-6-benzotriazolyl)phenol, 2-(2-hydroxy-4-octoxyphenyl)-4,6-bis(2,4-dimethylphenyl)-s-triazine, 2-(2-hydroxy-4-hexyloxyphenyl)-4,6-diphenyl-s-triazine, 2-(2-hydroxy-4-octoxyphenyl)-4,6-bis(2,4-dimethylphenyl)-s-triazine, 2-(2-hydroxy-4-hexyloxyphenyl)-4,6-diphenyl-s-triazine, 2-(2-hydroxy-4-propoxy-5-methylphenyl)-4,6-bis(2,4-dimethylphenyl)-s-triazine, 2-(2-hydroxy-4-hexyloxyphenyl)-4,6-dibiphenyl-s-triazine, 2,4-bis(2-hydroxy-4-octoxyphenyl)-6-(2,4-dimethylphenyl)-s-triazine, 2,4,6-tris(2-hydroxy-4-octoxyphenyl)-s-triazine, 2-hydroxybenzophenone, 2,4-dihydroxybenzophenone, 2-hydroxy-4-methoxybenzophenone, 2-hydroxy-4-octoxybenzophenone, and 5,5'-methylenebis(2-hydroxy-4-methoxybenzophenone).

The compounds disclosed in US 10,894,873 B2 and US 10,717,714 B2 can also be used as the ultraviolet absorbers, the contents of which are incorporated herein by entirety.

The examples of infrared absorbers include but is not limited to pentamethine cyanine derivatives, such as pentamethine benzoindolium compounds, pentamethine benzoxazolium compounds, and pentamethine benzothiazolium compounds; heptamethine cyanine derivatives, such as heptamethine indolium compounds, heptamethine benzoindolium compounds, heptamethine oxazolium compounds, heptamethine benzoxazolium compounds, heptamethine thiazolium compounds, and heptamethine benzothiazolium compounds; diimmonium compounds, aminium compounds, squarylium derivatives; nickel complexes, such as bis(stilbenedithiolato) compounds, bis(benzenedithiolato)nickel compounds, and bis(camphordithiolato)nickel compounds; azo dye derivatives, phthalocyanine derivatives, porphyrin derivatives, and dipyromethene metal chelate compounds.

The surface of the cured polymer can be treated by various ways, for example, chemical treatment, mechanical treatment, a corona discharge treatment, a flame treatment, a UV irradiation treatment, a radiofrequency treatment, a glow discharge treatment, an active plasma treatment, a laser treatment, a acid solution treatment, and an ozone oxidation treatment, as well as other treatments.

In one embodiment, the blue-violet light absorber is added in a specific ratio to the blue-violet light absorbing composition. For example, the amount of the blue-violet light absorber is about 0.01% to about 20%, preferably about 0.05% to about 10%, more preferably about 0.1% to about 5%, of the total weight of the composition.

In a particular embodiment, the blue-violet light absorbing composition include: about 0.01% to about 20%, preferably about 0.05% to about 10%, more preferably about 0.1% to about 5% of the blue-violet light absorber, and about 80% to about 100%, preferably about 90% to about 100%, more preferably about 95% to about 100% of the polymer. As used herein, the term "about 100% of the polymer" refers to the blue-violet light absorbing composition comprising extremely low amount of the blue-violet light absorber, for example, only comprising 0.01% to 0.5% of the blue-violet light absorber, or only comprising 0.01%, 0.05%, 0.1%, or 0.5% of the blue-violet light absorber. As used herein, the percentage (%) only refers to the total amount of the polymer and the blue-violet light absorber, without other additives.

Another aspect of the present disclosure provides a method of preparing a blue-violet light absorbing composition, comprising, mixing a polymer and a blue-violet light absorber, wherein the blue-violet light absorber is a compound of Formula (I) according to the present disclosure.

In one embodiment, the method further comprising: heating the polymer to about 200 °C to about 280 °C for melting, and cooling for curing. In a particular embodiment, the melting temperature of the polymer is about 200 °C, 210 °C, 220 °C, 230 °C, 240 °C, 250 °C, 260 °C, 270 °C, or 280 °C.

In a particular embodiment, the method further comprising: providing about 0.01 to about 20 parts by weight, preferably about 0.05 to about 10 parts by weight, more preferably about 0.1 to about 5 parts by weight of the blue-violet light absorber, and about 80 to about 100 parts by weight, preferably about 90 to about 100 parts by weight, more preferably about 95 to about 100 part by weights of the polymer, and mixing the blue-violet light absorber and the polymer to obtain the blue-violet light absorbing composition.

Another aspect of the present disclosure provides use of the blue-violet light absorbing composition according to the present disclosure in preparation of a blue-violet light absorbing product.

Accordingly, another aspect of the present disclosure provides a blue-violet light absorbing product comprising the blue-violet light absorbing composition according to the present disclosure.

In some embodiment, the blue-violet light absorbing product is selected from plastics, coating material, ink, and sunscreen.

In some embodiment, the blue-violet light absorbing product is selected from display device, lighting device, optical film, optical lens, glasses (e.g., goggle or contact lens), textile, and pressure sensitive adhesive.

In some embodiment, the blue-violet light absorbing product is an anti-blue-violet light and/or anti-UV lens or goggle, comprising glasses or high molecular material lens, such as polycarbonate, polymethylmethacrylate (PMMA), nylon (PA), TPX (Polymethylpentene), polystyrene, or poly(diethylene glycol bis(allyl carbonate)) (PEDC).

The blue-violet light absorbing composition of the present disclosure can be a light-absorbing layer or a light absorbing film in the product, the thickness of which varies according to the property of absorption or the disposition in the product, preferably being 0.1 µm to 100 µm. If the layer or film is too thin, the capability of light absorption may be insufficient; on the contrary, if the layer or film is too thick, it may result in irregular surface, uneven absorption, or fracture or wrinkle during heat processing.

Hence, in some embodiments, the blue-violet light absorbing product comprises a display device comprising a light-absorbing layer or light absorbing film, the blue-violet light of which is absorbed by the blue-violet light absorbing composition of the present disclosure. The display device includes but not limited to, for example, liquid crystal display (LCD), plasma display panel (PDP), electroluminescence device (ELD), cathode ray tube device (CRT), fluorescence display tube, and field emission display tube. When applying to a display device, the light-absorbing layer or light absorbing film is generally disposed in the front of the display device. For example, the light-absorbing layer or light absorbing film is directly disposed on the surface of the display device. If a front panel or a electromagnetic screen is disposed in the front of the display, the light-absorbing layer or light absorbing film can be adhered to the front (outer side) or the rear (display device side) of the front panel or electromagnetic screen.

Another aspect of the present disclosure provides a method of preparing blue-violet light absorbing product, wherein the blue-violet light absorbing product comprises the blue-violet light absorbing composition of the present disclosure.

In one embodiment, the method of preparing blue-violet light absorbing product comprises: providing the blue-violet light absorbing composition of the present disclosure, and disposing the blue-violet light absorbing composition on an article to obtain the blue-violet light absorbing product.

In another embodiment, the method of preparing blue-violet light absorbing product further comprises: mixing a polymer and a blue-violet light absorber according to the present disclosure to obtain a blue-violet light absorbing composition.

In some embodiment, the blue-violet light absorbing composition according to the present disclosure is processed in advance, for example, the blue-violet light absorbing composition is granulated or spun first, or is granulated before being spun.

In some embodiment, the blue-violet light absorbing composition according to the present disclosure is processed so as to disposed on the article to obtain the blue-violet light absorbing product. The method of processing includes but not limited to dip coating, extrusion, lamination, injection molding, calendaring, casting, film blowing, coating, melt blowing, or any combination thereof.

### Examples

### Preparation of compound

### Comparative Example 1: Ethyl-2-cyano-3-(4-(dimethylamino)phenyl) acrylate

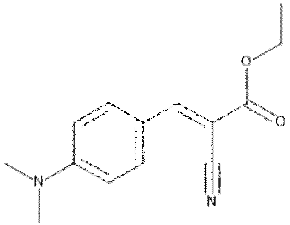

### Ethyl-2-cyano-3-(4-(dimethylamino)phenyl)acrylate

The synthesis of ethyl-2-cyano-3-(4-(dimethylamino)phenyl)acrylate: 15g 4-(dimethylamino)benzaldehyde and 14.5 g 2-cyano ethyl acetate were dissolved in dichloromethane and was stirred. Molecular sieve was added to remove water and a calcium chloride tube was disposed to prevent from water. 1 ml piperidine and 0.6 ml acetic acid were then added. The solution was heated and refluxed for two hours while supplementing fresh molecular sieve during reaction. The solvent was removed after completion of reaction, and after washing with acid and drying, ethyl-2-cyano-3-(4-(dimethylamino)phenyl)acrylate was obtained. Yield: 95%; melting point: 124-127 °C; UV-Vis(CH₃CN max.): 418 nm; 10% loss of TGA in the atmosphere: 235°C.

### Comparative Example 2: Ethyl-2-cyano-3-(4-(diethylamino)phenyl)acrylate

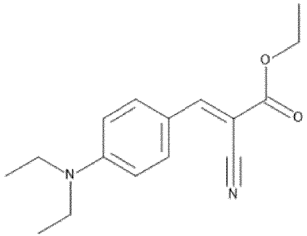

### Ethyl-2-cyano-3-(4-(diethylamino)phenyl)acrylate

The synthesis of ethyl-2-cyano-3-(4-(diethylamino)phenyl)acrylate: 328g diethylaminobenzaldehyde, 251g cyano ethyl acetate, and 500g ethanol were put into a reaction flask purged with nitrogen. 18.7g triethylamine was added dropwise at 50 °C and heated at 70 °C for 2.5 hours. After cooling to room temperature, the precipitated solid after filtration was washed with ethanol, and then dried under reduced pressure to obtain ethyl-2-cyano-3-(4-(diethylamino)phenyl)acrylate. Orange solid. Yield: 93%; melting point: 96-97 °C; UV absorption peak: 421 nm; 10% loss of TGA in the atmosphere: 252°C.

### Comparative Example 3: Methyl-2-cyano-3-(4-(diethylamino)phenyl) acrylate

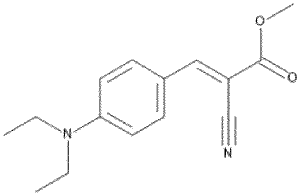

### Methyl-2-cyano-3-(4-(diethylamino)phenyl)acrylate

The synthesis of methyl-2-cyano-3-(4-(diethylamino)phenyl)acrylate: 328g diethylaminobenzaldehyde, 220g cyano methyl acetate, and 500g ethanol were put into a reaction flask purged with nitrogen. 18.7g triethylamine was added dropwise at 50 °C and heated at 70 °C for 2.5 hours. After cooling to room temperature, the precipitated solid after filtration was washed with ethanol and then dried under reduced pressure to obtain methyl-2-cyano-3-(4-(diethylamino)phenyl)acrylate. Orange solid. Yield: 81%; melting point: 87-92 °C; UV absorption peak: 423 nm; 10% loss of TGA in the atmosphere: 235°C.

### Example 1: 3-[4-(dimethylamino)phenyl]-2-(morpholinyl-4-carbonyl)prop-2-enenitrile

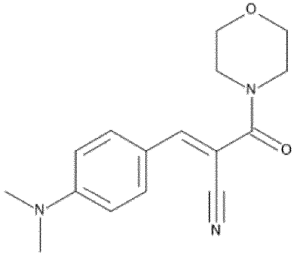

### 3-[4-(dimethylamino)phenyl]-2-(morpholinyl-4-carbonyl)prop-2-enenitrile

The synthesis of 3-[4-(dimethylamino)phenyl]-2-(morpholinyl-4-carbonyl)prop-2-enenitrile: 10g ethyl-2-cyano-3-(4-(dimethylamino)phenyl)acrylate (Comparative Example 1) and 40g morpholine were mixed at 130 °C to react for 8.0 hours, followed by cooling and filtering the solid. Then, the filter cake was washed with methanol and dried to obtain 3-[4-(dimethylamino)phenyl]-2-(morpholinyl-4-carbonyl)prop-2-enenitrile. Yellow solid. Yield: 67%; purity: more than 98.8%; melting point: 112-118 °C; UV absorption peak: 403nm; 10% loss of TGA in the atmosphere: 298 °C.

### NMR analysis:

1H NMR (CDCl3, 300 MHz) (Chemical shift of the peak, ppm; multiplicity; number of protons): 3.08(s, 6H), 3.70-3.75 (m, 4H), 6.69(d, 2H),, 7.72(s, 1H), 7.86(d, 2H) 13C NMR (CDCl3, 125 MHz): 40.0, 44.5, 46.1, 66.7, 96.8, 111.5, 118.2, 120.1, 133.0, 134.2, 153.1, 153.7, 165.1

### Example 2: 3-[4-(diethylamino)phenyl]-2-(morpholinyl-4-carbonyl)prop-2-enenitrile

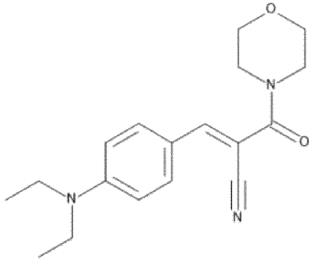

### 3-[4-(diethylamino)phenyl]-2-(morpholinyl-4-carbonyl)prop-2-enenitrile

The synthesis of 3-[4-(diethylamino)phenyl]-2-(morpholinyl-4-carbonyl)prop-2-enenitrile: 10g ethyl-2-cyano-3-(4-(diethylamino)phenyl)acrylate (Comparative Example 2) and 40g morpholine were mixed at 130 °C to react for 8.0 hours, followed by cooling and filtering the solid. Then, the filter cake was washed with methanol and dried to obtain 3-[4-(diethylamino)phenyl]-2-(morpholinyl-4-carbonyl)prop-2-enenitrile. Yellow solid. Yield: 28%; purity: more than 99%; melting point: 88-90 °C; UV absorption peak: 403nm; 10% loss of TGA in the atmosphere: 291 °C.

### NMR analysis:

1H NMR (CDCl3, 300 MHz): 1.22 (t, 3H), 3.40-3.47(q, 4H), 3.72(d, 8H), 6.68(d, 2H), 7.71(s, 1H), 7.84(d, 2H)
13C NMR (CDCl3, 125 MHz): 12.5, 44.7, 46.3, 66.7, 95.9, 111.2, 118.3, 119.5, 133.3, 150.9, 153.5, 165.2

### Example 3: 3-[4-(diethylamino)phenyl]-2-(pyrrolidinyl-4-carbonyl)prop-2-enenitrile

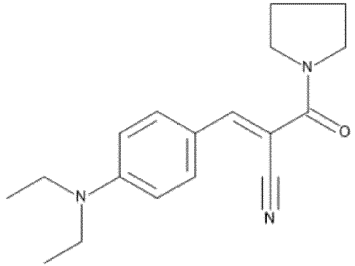

### 3-[4-(diethylamino)phenyl]-2-(pyrrolidinyl-4-carbonyl)prop-2-enenitrile

The synthesis of 3-[4-(diethylamino)phenyl]-2-(pyrrolidinyl-4-carbonyl)prop-2-enenitrile: 10g ethyl-2-cyano-3-(4-(diethylamino)phenyl)acrylate (Comparative Example 2) and 40g pyrrolidine were mixed at 90 °C to react for 24 hours, followed by cooling and filtering the solid. Then, the filter cake was washed with methanol and dried to obtain 3-[4-(diethylamino)phenyl]-2-(pyrrolidinyl-4-carbonyl)prop-2-enenitrile. Yield: 76%; purity: more than 99%; melting point: 112-118 °C; UV absorption peak: 405nm; 10% loss of TGA in the atmosphere: 285 °C.

### NMR analysis:

1H NMR (CDCl3, 300 MHz): 1.20(t, 4H), 1.91-1.94(m, 4H), 3.40-3.45(m, 6H), 3.58(t ,2H), 3.77(t,2H), 6.65(d, 2H), 7.86(d, 2H), 7.90(s, 1H)
13C NMR (CDCl3, 125 MHz): 12.6, 24.2, 26.8, 44.8, 47.6, 48.7, 97.2, 111.1, 118.6, 119.6, 133.5, 150.9, 153.5, 163.3

### Example 4: 3-[4-(dimethylamino)phenyl]-2-(pyrrolidinyl-4-carbonyl)prop-2-enenitrile

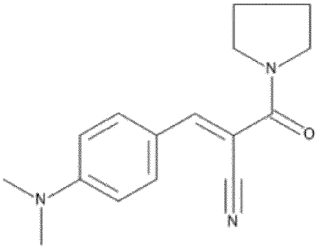

### 3-[4-(dimethylamino)phenyl]-2-(pyrrolidinyl-4-carbonyl)prop-2-enenitrile

The synthesis of 3-[4-(dimethylamino)phenyl]-2-(pyrrolidinyl-4-carbonyl)prop-2-enenitrile: 10 g ethyl-2-cyano-3-(4-(dimethylamino)phenyl)acrylate (Comparative Example 1) and 40g pyrrolidine were mixed at 90 °C to react for 24 hours, followed by cooling and filtering the solid. Then, the filter cake was washed with methanol and dried. After reaction for 8.0 hours, the solid was cooled and filtered. Then, the filter cake was washed with methanol and dried to obtain 3-[4-(dimethylamino)phenyl]-2-(pyrrolidinyl-4-carbonyl)prop-2-enenitrile. Yield: 90%; purity: more than 99%; melting point: 118-128 °C; UV absorption peak: 395nm; 10% loss of TGA in the atmosphere: 281 °C.

### NMR analysis:

1H NMR (CDCl3, 300 MHz): 1.94 (m, 4H), 3.08 (s, 6H), 3.59 -3.78 (m, 4H), 6.68(d, 2H), 7.87 (s, 1H),7.91 (d, 2H)
13C NMR (CDCl3, 125 MHz): 24.2, 26.8, 40.0, 47.6, 48.7, 98.1, 111.5, 118.4, 120.2, 131.7, 120.2, 131.7, 133.1, 153.0, 153.5, 163.1

### Functional Assay

### Thermostability Analysis

The blue-violet light absorber requires high temperature processing when being added in plastics, or is used outdoors under high temperature. Nevertheless, general anti-blue-violet light agent is unable to resist high temperature. Thus, high stability under high temperature is a very essential requirement for blue-violet light absorbers. The melting temperature of polycarbonate (PC) is about 250-270 °C, and hence in the condition of high temperature processing of plastics, a blue-violet light absorber has a TGA (thermogravimetric analysis) change less than 10%, which can effectively avoid thermal degradation or decomposition of the blue-violet light absorber due to high temperature during producing and processing and thereby prevent the loss of blue-violet light absorption function. The thermostability of the compounds of Examples 1 to 4 and the compounds of Comparative Examples 1-3 were determined by thermogravimetric analyzer (TGA) and were tested for the temperature of 10% thermogravimetric loss, in which the higher temperature represents better thermostability.

The results were shown in Table 1. The blue-violet light absorbers of Comparative Examples 1 and 3 have a 10% thermogravimetric loss of TGA at the temperature of 235 °C, which cannot be used in thermal processing of polycarbonate (PC). The blue-violet light absorber of Comparative Example 2 has a 10% thermogravimetric loss of TGA at the temperature of 252 °C, which also has poor thermostability. The compound of Example 1 of the present disclosure has only 10% thermogravimetric loss when the temperature was elevated to 292°C, which is excellent thermostability and can be used in the thermal processing of polycarbonate (PC). In addition, the temperature of 10% thermogravimetric loss of TGA in Examples 2, 3, and 4 is 291°C, 285°C, and 281°C, respectively, which are all superior to Comparative Examples 1 to 3 and thus can be used in the thermal processing of plastics.

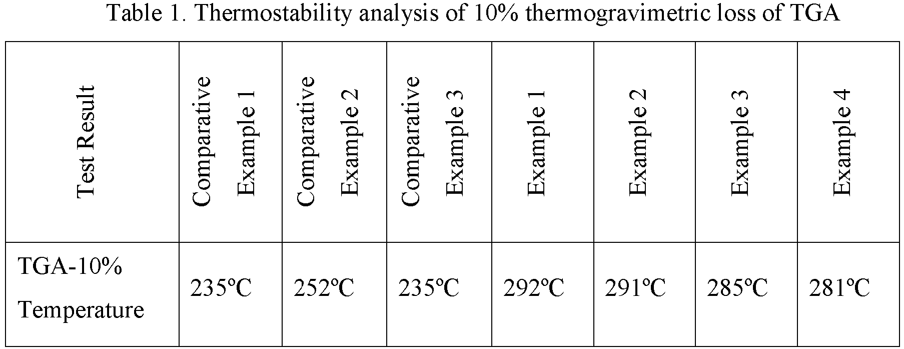

### Analysis of blue-violet light absorption

The wavelength of blue-violet light has a range from 380 to 460 nm, in which the shorter wavelength has higher energy and causes more photochemical damage to retina. Therefore, more absorption of a blue-violet light absorber for the blue-violet light with shorter wavelength at from 380 to 420 nm (higher energy) means better protective effect from the blue-violet light with shorter wavelength. The maximal absorbance peaks of the compounds of Examples 1 to 4 and Comparative Examples 1 to 3 were measured by a UV-Vis spectrophotometer.

### Maximal absorbance peak assay

The UV-Vis spectrophotometer (Type: Varian Cary^{®} 50, purchased from Agilent) was used to test the maximal absorbance peak of a sample (λmax, nm).

The results were shown in Table 2. The blue-violet light absorbers of Comparative Examples 1 to 3 have maximal absorbance peak at 418nm, 421nm, and 423nm respectively, in which the peak of maximal absorbance is at the blue-violet light with a longer wavelength. The blue-violet light absorbers of Examples 1 to 4 of the present disclosure have maximal absorbance peak at 403nm, 403nm, 405nm, and 395nm respectively, in which the peak of maximal absorbance is at the blue-violet light with shorter wavelength, indicating the improved protective effect from the blue-violet light with a shorter wavelength.

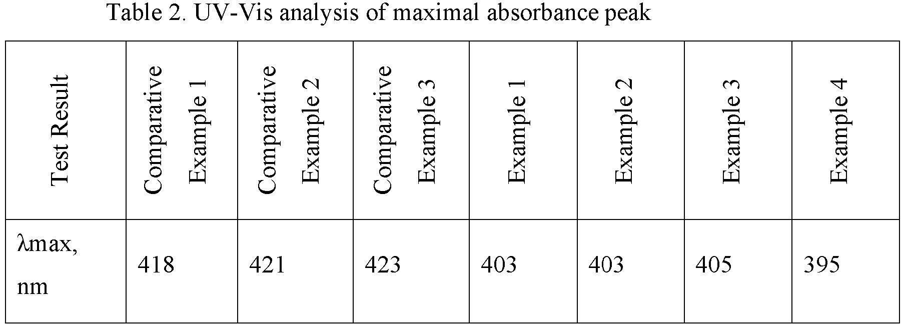

### Test of lifetime of anti-blue-violet light composition and anti-blue-violet light film

30 g CY499 resin comprising 1 wt% the blue-violet absorber and 5.5 g HDT-90B curing agent were coated on PET and prepared as a coating film having a dry film thickness of 50 µm, which was tested for weather resistance using a xenon lamp (ASTM G155). Measurement and observation was continuously carried out over time for the shortest wavelength of transmittance (T%)= 1%.

### Weathering Resistance Test -Xenon lamp

The sample was disposed under a xenon lamp aging tester (Type: Q-SUN Xenon Test Chamber, purchased from Q-Lab), and the weather resistance test is carried out under the following condition: black panel temperature: 60 °C; irradiance: 0.5 W/m² @340 nm.

Table 3 discloses the 1% transmittance wavelength of the anti-blue-violet light film comprising the blue-violet light absorbers according to Comparative Examples 1 to 3 and Examples 1 to 4 and the negative control without a blue-violet light absorber.

The compounds of the Examples were tested at 0 hour for 1% transmittance wavelength as follows: Example 1: 442nm; Example 2: 448nm; Example 3: 448nm; Example 4: 435nm. Compared to Comparative Examples 1 to 3, Examples 1 to 4 absorbed the blue-violet light with shorter wavelength. After irradiation of the xenon lamp for 117 hours, 1% transmittance wavelength of Comparative Example 1 and Comparative Example 2 was declined to 316 nm and 315 nm, which has lost the function of blue-violet light absorption. After irradiation of the xenon lamp for 134 hours, 1% transmittance wavelength of Comparative Example 3 was declined to 315 nm, which has lost the function of blue-violet light absorption. In contrast, after irradiation of the xenon lamp for 134 hours, 1% transmittance wavelength of Example 1 to 4 according to the present disclosure is 393 nm, 413 nm, 411 nm, and 398 nm respectively, which indicates that the Examples of the present disclosure still maintained excellent blue-violet light protective effect after long-term irradiation of the xenon lamp.

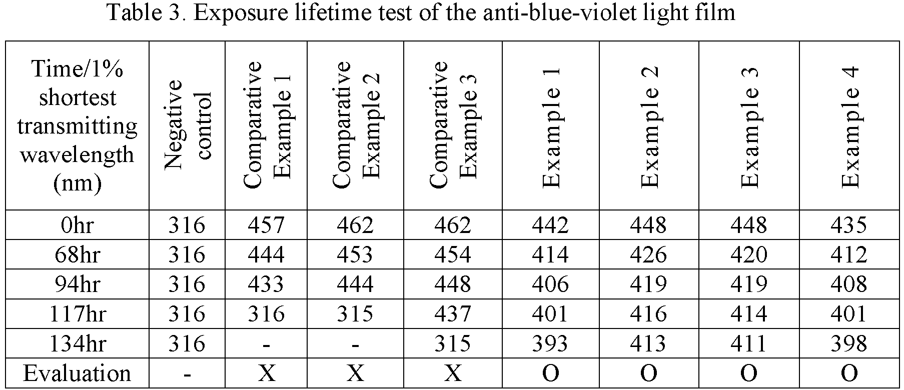

According to the present disclosure, the compound of Formula (I) can be mixed with another polymer and used in the preparation of blue-violet light absorbing products. The application of the products includes, but not limited to, the technical field such as plastics, coating materials, ink, display devices, lighting devices, optical films, optical lens, goggles, glasses, textiles, pressure sensitive adhesives, or sunscreen products; particularly in the screen protecting films of mobile phones, computers, or televisions, or in glasses industry and thus have potential application prospects .

In view of the Examples disclosed in the specification, a skilled person in the field of present disclosure would readily understand that the foregoing Examples are provided by way of examples rather than limiting. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and their equivalents be covered thereby.

## Claims

1. A blue-violet light absorbing composition, comprising a polymer and a blue-violet light absorber, wherein the blue-violet light absorber is a compound of the following Formula (I): 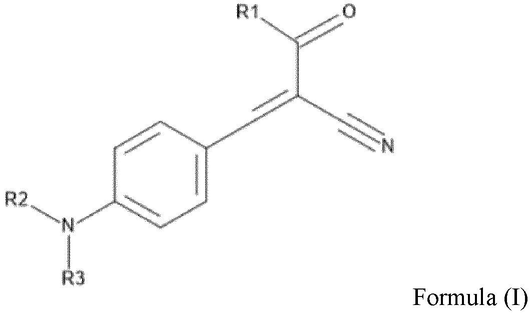
wherein R₁ is a five-membered or six-membered heterocycloalkyl group comprising 1 or 2 heteroatom(s) selected from the group consisting of oxygen and nitrogen and is unsubstituted or substituted with at least one of C₁₋₈ alkyl, -OH, -N=O, -CN, or halogen; and
R₂ and R₃ are independently selected from H or C₁₋₈ alkyl.

2. The blue-violet light absorbing composition of claim 1, wherein R₁ is selected from the group consisting of unsubstituted or substituted pyrrolidinyl, tetrahydrofuranyl, oxazolidinyl, isoxazolidinyl, tetrahydropyranyl, piperidinyl, morpholinyl, 1,2-oxazinanyl, and 1,3-oxazinanyl.

3. The blue-violet light absorbing composition of claim 1, wherein
R₁ is selected from 4-morpholinyl, 1-piperidinyl, or pyrrolidin-1-yl;
R₂ is selected from H, methyl, or ethyl; and
R₃ is selected from H, methyl, or ethyl.

4. The blue-violet light absorbing composition of claim 1, wherein the blue-violet light absorber is selected from one or any combination of the following compounds :

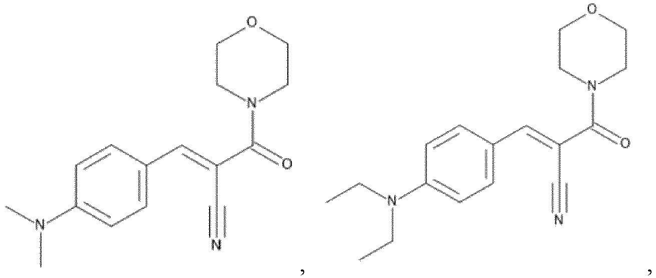

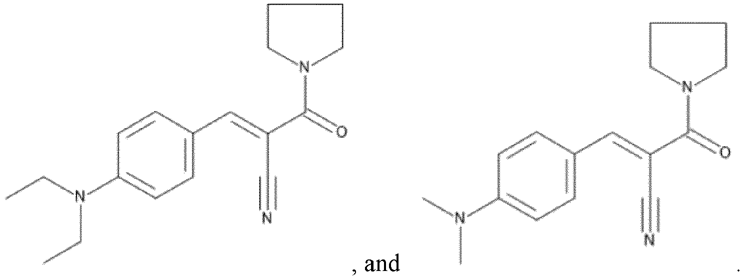

5. The blue-violet light absorbing composition of claim 1, wherein the polymer is a curable transparent or semitransparent polymer.

6. The blue-violet light absorbing composition of claim 1, wherein the polymer is selected from the group consisting of cellulose ester, polyamide, polyimide, polyurethane, epoxy resin, amino resin, polycarbonate, polyester, polyolefin, acrylic resin, polyformaldehyde, polysulfone, polyethersulfone, polyesterketone, polyether imide, polyoxyethylene, silicone, liquid crystal polymer, and any combination thereof.

7. The blue-violet light absorbing composition of claim 1, further comprising one or more additional additive selected from the group consisting of antistatic agent, antifoaming agent, levelling agent, wetting agent, thickening agent, dispersing agent, wax, matting agent, antimicrobial agent, metal oxide screening agent, photostabilizing agent, thermostabilizing agent, antioxidant, peroxide scavenger, free radical scavenger, filler, rubber, preservative, fire retardant, plasticizer, dye, pigment, brightener, fluorescent whitening agent, antiager, metal stabilizer, acid scavenger, antihydrolysis agent, and any combination of at least two of the aforesaid additives.

8. The blue-violet light absorbing composition of claim 1, further comprising one or more light absorber selected from the group consisting of infrared absorber, ultraviolet absorber, blue light absorber, and any combination of at least two of the aforesaid absorbers.

9. The blue-violet light absorbing composition of claim 1, wherein the amount of the blue-violet light absorber is 0.01% to 20% of the total weight of the blue-violet light absorbing composition.

10. A method of preparing a blue-violet light absorbing composition, comprising mixing a polymer and a blue-violet light absorber to obtain a blue-violet light absorbing composition, wherein the blue-violet light absorber is a compound of the following Formula (I): 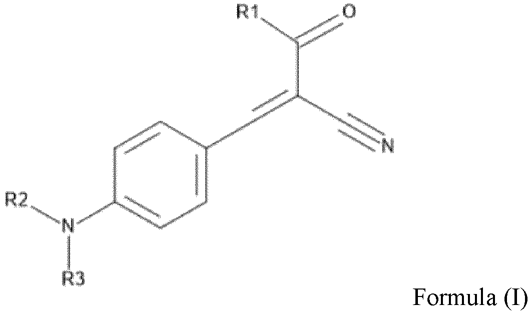
wherein R₁ is a five-membered or six-membered heterocycloalkyl group comprising 1 or 2 heteroatom(s) selected from the group consisting of oxygen and nitrogen and is unsubstituted or substituted with at least one of C₁₋₈ alkyl, -OH, -N=O, -CN, or halogen;
and
R₂ and R₃ are independently selected from H or C₁₋₈ alkyl.

11. The method of claim 10, wherein R₁ is selected from the group consisting of unsubstituted or substituted pyrrolidinyl, tetrahydrofuranyl, oxazolidinyl, isoxazolidinyl, tetrahydropyranyl, piperidinyl, morpholinyl, 1,2-oxazinanyl, and 1,3-oxazinanyl.

12. The method of claim 10, wherein
R₁ is selected from 4-morpholinyl, 1-piperidinyl, or pyrrolidin-1-yl;
R₂ is selected from H, methyl, or ethyl; and
R₃ is selected from H, methyl, or ethyl.

13. The method of claim 10, wherein the blue-violet light absorber is selected from one or any combination of the following compounds:

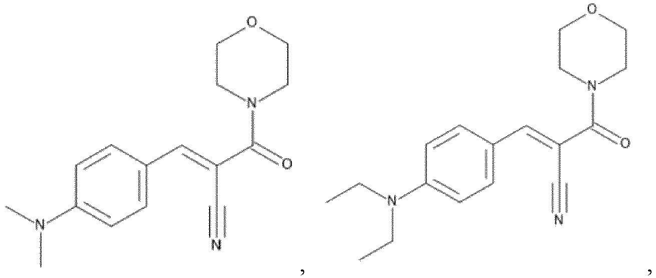

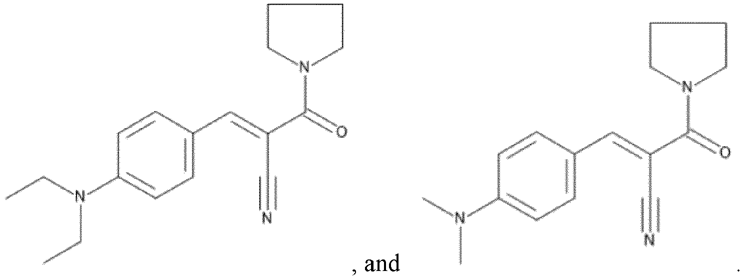

14. The method of claim 10, wherein 0.01 to 20 parts by weight of the blue-violet light absorber is mixed with 80 to 100 parts by weight of the polymer.

15. The method of claim 10, further comprising: heating the polymer to 200 °C to 280 °C for melting, and cooling for curing.

16. A blue-violet light absorbing product, comprising the blue-violet light absorbing composition of claim 1 to 9.

17. The blue-violet light absorbing product of claim 16, selected from the group consisting of plastics, coating material, ink, sunscreen, display device, lighting device, optical film, optical lens, glasses, textile, and pressure sensitive adhesive.

18. A method of preparing blue-violet light absorbing product, comprising:
providing the blue-violet light absorbing composition of claim 1 to 9, and
disposing the blue-violet light absorbing composition on an article to obtain the blue-violet light absorbing product.

19. The method of claim 18, wherein the blue-violet light absorbing composition is processed via a method selected from the group consisting of dip coating, granulation, extrusion, lamination, injection molding, calendaring, casting, film blowing, coating, melt blowing, fagoting, or any combination thereof.
